Europäisches Patentamt

European Patent Office

Office européen des brevets

(19)

(11) Numéro de publication : **0 376 852 B1**

(12) **FASCICULE DE BREVET EUROPEEN**

(45) Date de publication du fascicule du brevet :
23.06.93 Bulletin 93/25

(51) Int. Cl.⁵ : **A61K 9/16, A61K 47/14**

(21) Numéro de dépôt : **89403675.5**

(22) Date de dépôt : **28.12.89**

(54) **Microgranulés chargés en une substance active, constitués essentiellement d'un triglycéride d'acides gras saturés et leur utilisation par voie topique dans le domaine thérapeutique.**

(30) Priorité : **29.12.88 LU 87416**

(43) Date de publication de la demande :
**04.07.90 Bulletin 90/27**

(45) Mention de la délivrance du brevet :
**23.06.93 Bulletin 93/25**

(84) Etats contractants désignés :
**AT BE CH DE ES FR GB GR IT LI NL SE**

(56) Documents cités :
**DE-A- 2 725 924**
**FR-A- 2 218 086**
**GB-A- 543 309**

(73) Titulaire : **CENTRE INTERNATIONAL DE RECHERCHES DERMATOLOGIQUES GALDERMA - CIRD GALDERMA**
**Sophia Antipolis**
**F-06560 Valbonne (FR)**

(72) Inventeur : **Jamoulle, Jean-Claude**
**52, allée de la Grange**
**F-06250 Mougins (FR)**
Inventeur : **Allec, Josiane Les Vergers de Val Constance**
**300, Chemin de la Suquette**
**F-06600 Antibes (FR)**
Inventeur : **Brzokewicz, Alain**
**Les Jonquilles Batiment 4 Les Semboules**
**F-06600 Antibes (FR)**
Inventeur : **Shroot, Braham**
**Villa 35 Hameaux de Val Bosquet**
**Chemin de Val Bosquet F-06600 Antibes (FR)**

(74) Mandataire : **Nony, Michel et al**
**Cabinet NONY & CIE 29, rue Cambacérès**
**F-75008 Paris (FR)**

## Description

La présente invention a pour objet des microgranulés chargés en une substance active constitués par un triglycéride d'acides gras saturés et leur utilisation par voie topique dans des compositions pharmaceutiques.

Un grand nombre de substances actives ne peuvent être formulées en vue d'une application topique du fait de leur manque de stabilité physique ou chimique, soit vis-à-vis du milieu de formulation, soit vis-à-vis des conditions de stockage en particulier vis-à-vis de l'oxygène atmosphérique.

La stabilisation de substances actives dans des formulations à usage pharmaceutique, et l'incorporation de substances actives chimiquement incompatibles dans une même formulation ainsi d'ailleurs que la libération progressive de la (ou des) substance(s) active(s) à partir de formulations, sont trois problèmes importants qui se posent au galéniste.

Jusqu'à présent, la stabilisation des substances actives a été résolue par exemple par la technique de microencapsulation consistant à former à l'aide d'un polymère approprié des microcapsules de la substance active. Cette technique présente, cependant, lorsque les compositions sont destinées à une application topique, un certain inconfort en abandonnant sur la peau l'enveloppe résiduelle de la microcapsule, ce qui procure un aspect rugueux à la composition lors de son application.

Par ailleurs l'emploi de microcapsules ne permet pas toujours une libération progressive de la substance active dans de bonnes conditions.

Dans le brevet français n°2.581.541 ayant le numéro d'enregistrement national 85.07013, il a été proposé des microparticules constituées d'un principe actif, d'un polymère biodégradable ou non et d'un excipient lipidique permettant la libération prolongée du principe actif.

Dans ces microparticules, le polymère joue le rôle d'un agent structurant et l'excipient lipidique a pour objet d'empêcher la pénétration des liquides biologiques afin de ralentir la libération du principe actif.

Ces microparticules si elles permettent effectivement une libération prolongée du principe actif présentent également le même inconvénient que les microcapsules dans la mesure où elles sont constituées d'un polymère. Par ailleurs l'excipient lipidique utilisé est généralement d'un point de fusion élevé de telle sorte que les microparticules confèrent aux compositions un aspect granuleux sans effet lubrifiant pour la peau ou les muqueuses.

La présente invention apporte une solution nouvelle et satisfaisante quant à la stabilisation et à la libération progressive et localisée de substances actives.

La présente invention a, en effet, pour objet des microgranulés chargés en une substance active, fondant par application sur la peau en libérant ladite substance active, ceux-ci étant essentiellement formés à l'aide d'un triglycéride d'acides gras saturés présentant un point de fusion inférieur à 50°C.

L'utilisation de triglycérides d'acides gras saturés présente l'avantage sur les triglycérides d'acides gras insaturés d'être stables à l'oxydation et sur les mono et di-glycérides de ne pas contenir de groupement hydroxyle résiduel sur le squelette glycérol et dès lors de conserver une lipophilie maximale favorable à la suspension dans les phases hydrophiles sans pseudo-solubilisation.

Les différentes études qui ont été réalisées ont permis de montrer que les substances actives incorporées dans les microgranulés étaient parfaitement stables sur de longues périodes de temps.

Ce type de formulation de substances actives permet ainsi d'associer dans une même composition diverses substances actives incompatibles, l'une des substances actives pouvant se trouver incorporée dans les microgranulés et l'autre dans la phase dispersante ou encore les deux substances actives peuvent se trouver respectivement incorporées dans des lots différents de microgranulés, eux-mêmes dispersés dans une phase hydrophile ou encore parmi des microgranulés non chargés en substance active.

L'utilisation de triglycérides d'acides gras saturés, ayant un point de fusion inférieur à 50°C, pour préparer ces microgranulés a pour effet principal que lorsque la formulation contenant les microgranulés vient au contact de la peau, de préférence avec un léger massage, ceux-ci fondent progressivement en libérant la substance active et en abandonnant sur la peau un film lipidique, l'ensemble contrôlant la diffusion de la substance dans les couches cutanées.

La présente invention a donc pour objet, à titre de produit industriel nouveau, des microgranulés chargés en une substance thérapeutiquement active sur les désordres cutanés et fondant par application sur la peau, ceux-ci étant essentiellement formés, par granulation de ladite substance active, à l'aide d'au moins un triglycéride d'acides gras saturés, linéaires ou ramifiés, ayant de 10 à 22 atomes de carbone, ayant un point de fusion compris entre 30 et 50°C, lesdits microgranulés ayant un diamètre moyen compris entre environ 5$\mu$m et 800$\mu$m mais de préférence entre 50$\mu$m et 500$\mu$m.

Par l'expression "microgranulés" on doit entendre des microparticules ou microsphères pleines et non creuses.

Les acides gras des triglycérides proviennent de préférence d'acides gras végétaux naturels.

Les acides gras des triglycérides proviennent d'acides gras végétaux naturels, saturés et linéaires ou ramifiés ayant de 10 à 22 atomes de carbone.

Le point de fusion des triglycérides doit être inférieur à 50°C et de préférence compris entre 30 et 50°C.

Leur indice d'hydroxyle doit être inférieur ou égal à 15 et leur indice d'iode inférieur ou égal à 3.

Parmi ces triglycérides d'acides gras tels que définis ci-dessus ceux particulièrement préférés, selon l'invention, sont ceux vendus sous la dénomination de "SOFTISAN®" par la Société DYNAMIT NOBEL. Parmi ceux-ci on peut notamment citer les suivants:

- "SOFTISAN 100®" de point de fusion 33-36°C
- "SOFTISAN 133®" de point de fusion 32-34°C
- "SOFTISAN 138®" de point de fusion 37-39°C
- "SOFTISAN 142®" de point de fusion 42-44°C.

On peut également utiliser les triglycérides vendus sous la dénomination de "SUPPOCIRE®" par la Société GATTEFOSSE et parmi ceux-ci le "SUPPOCIRE DM®" de point de fusion 43-45°C et le "SUPPOCIRE CM®" de point de fusion 38-40°C.

De façon générale selon la présente invention, les microgranulés contiennent de 0,01 à 10% en poids de la substance active, le reste étant essentiellement constitué par le triglycéride ou un mélange de triglycérides.

Le point de fusion des microgranulés chargés en substance active varie en fonction du pourcentage et de l'état solide ou liquide de la substance active présente. Celui-ci n'est généralement différent que de quelques degrés seulement par rapport au point de fusion du ou des triglycérides utilisés pour la formation des microgranulés si la substance active est solide ou semi-solide à température ambiante.

La préparation des microgranulés peut être réalisée selon divers procédés de granulation, en particulier selon un procédé de dispersion à l'état fondu suivi d'une solidification. Selon ce procédé, la substance active et le ou les triglycéride(s), dans les proportions requises, sont mis à chauffer à une température d'environ 6°C au dessus du point de fusion du ou des triglycéride(s) puis placés dans un bain à ultrasons. Le mélange est ensuite versé dans de l'eau stérile à la même température sous agitation et la dispersion obtenue est alors rapidement refroidie par addition d'eau stérile froide sous agitation constante. Les microgranulés chargés en substance active sont alors filtrés et séchés à température ambiante et sous vide puis soumis à une opération de tamisage.

Les études qui ont été réalisées ont montré qu'à vitesse constante, la taille des particules avait tendance à décroître en fonction de l'augmentation du point de fusion du triglycéride utilisé.

Les microgranulés peuvent également être préparés selon un procédé d'atomisation à l'aide d'un ensemble de pulvérisation sous pression.

Dans un réacteur en acier inoxydable thermostaté, le ou les triglycéride(s) sont fondu(s) et le principe actif à associer est soit fondu, soit dispersé sous atmosphère d'azote, à l'abri de la lumière. Le mélange fondu est poussé par de l'azote (pression de 0,5 à 3 bars) jusqu'à la buse à un certain débit et l'atomisation est réalisée au niveau de la buse sous pression d'azote (pression de 1 à 10 bars).

La pulvérisation est réalisée dans une cuve en acier inoxydable étanche, possédant un gradient de température de bas en haut. Ce gradient, d'environ -150°C (bas) à 20°C (haut), peut être créé par introduction préalable d'azote liquide au fond de la cuve.

En fonction du type de buse choisi, la pression d'azote de pulvérisation et le débit du liquide conditionnent le diamètre moyen des microgranulés obtenus. Ainsi, en règle générale, plus le débit est faible, plus les gouttelettes en haut de la cuve et par conséquent, les microgranulés au bas de la cuve son-petits. D'autre part, plus la pression de pulvérisation est faible, plus les microgranulés ont un diamètre important et une distribution en taille étendue. Par rapport au procédé précédemment décrit, la non-utilisation d'un milieu aqueux peut s'avérer d'un grand intérêt par exemple lorsque l'on souhaite obtenir des microgranulés chargés en une substance active hydrosoluble.

Selon ces procédés, les microgranulés se présentent essentiellement sous forme de particules sphériques.

Bien que l'invention ne soit pas limitée à certaines substances actives, elle présente néanmoins un intérêt tout particulier à l'égard de substances peu stables en milieu aqueux.

Parmi les substances actives qui peuvent être incorporées par formation de microgranulés, on peut notamment mentionner sans que cette énumération soit limitative:

- la vitamine A, l'acide rétinoïque et ses dérivés, les vitamines E et F et leurs dérivés, l'acide 6- [3-(1-adamantyl-4) méthoxyphényl] naphtoïque-2 et ses analogues, l'acide 2-(5,6,7,8-tétrahydro-5,5,8,8- tétraméthyl-2-naphtyl)-6-benzo (b) thiophène carboxylique et ses analogues, l'acide 4- [3-(1-adamantyl)-4-méthoxybenzamido ] benzoïque et ses analogues, les corticostéroïdes tels que le 17-valérate de bétaméthasone ou le 17,21-dipropionate de bétaméthasone, les anti-inflammatoires non stéroïdiens tels que l'indométhacine, le bufexamac, le N-benzylphényl-αacétoxyacétamide ; ou d'autres dérivés du N-ben-

EP 0 376 852 B1

zylmandélamide tels que le nicotinate de N-benzylmandélamide, le benzoate de N-benzylmandélamide, l'acétate de N-(p-chloro) benzylmandélamide, l'acétate de N-(p-méthyl) benzylmandélamide, l'acétate de N-méthyl, N-benzylmandélamide, l'O-éthoxy-carbonyl-N-benzylmandélamide ; le N-phénéthyl-diphényl-acétoxyacétamide ; les dérivés d'acides gras polyinsaturés tels que l'acide éicosatriynoïque, les antihistaminiques tels que la prométhazine ou la cinnarizine, les antiprurigineux tels que l'acide palmitoyl-collagénique, les antifongiques tels que les dérivés d'éconazole ou les dérivés de pyrithione, les antipsoriasiques tels que l'anthraline et ses dérivés, les antibactériens tels que les sulfamides, les antibiotiques tels l'érythromycine, les antiseptiques tels que les parahydroxybenzoates de propyle ou de benzyle, les antiviraux et antiherpétiques tels que la vidarabine, les anesthésiques tels que la benzocaïne, les kératolytiques tels que l'acide salicylique ou les goudrons, ou les peroxydes tels que le peroxyde de benzoyle, les agents vasodilatateurs tels que les esters de l'acide nicotinique, les agents vasculoprotecteurs ou veinotoniques tels que l'extrait de marron d'inde, les agents anticouperose, les agents aidant à la cicatrisation, les agents anti-chute ou favorisant la repousse des cheveux, tels que le minoxidil etc...

L'incorporation de ces substances actives dans les microgranulés permet d'en améliorer de façon particulièrement significative la stabilité physique ou chimique.

On peut également incorporer dans les microgranulés des substances ayant la propriété de modifier les caractéristiques physico-chimiques cutanées. On peut citer à titre d'exemple, les substances émollientes ou les substances modifiant la perméabilité de la couche cornée ou la fluidité des lipides cutanés.

La présente invention a également pour objet des compositions pharmaceutiques notamment pour le traitement des désordres cutanés ou ophtalmologiques, contenant, dans un véhicule approprié, des microgranulés tels que définis ci-dessus.

Selon le véhicule compatible avec les microgranulés les compositions peuvent se présenter sous forme d'une lotion, d'un gel aqueux hydroalcoolique ou hydroglycolique, d'une formulation à base de silicone, d'une poudre (la charge étant de l'oxyde de zinc et/ou du talc) ou d'une pommade.

Parmi les agents épaississants utilisables pour la formation des gels, on peut notamment citer les polymères carboxyvinyliques tels que ceux vendus sous la dénomination de "CARBOPOL®" par la Société GOODRICH BF, notamment le "CARBOPOL 940®" ou le "CARBOPOL 930®" ou les dérivés de cellulose tels que l'hydroxypropylcellulose vendue sous la dénomination de "KLUCEL HF®" par la Société HERCULES.

Les microgranulés selon l'invention sont généralement présents en une proportion pouvant être comprise entre 1 et 50% en poids par rapport au poids total de la composition.

Comme ceci a été indiqué précédemment, ces compositions peuvent contenir des mélanges de microgranulés chargés séparément en substances actives incompatibles ou encore contenir dans la phase dispersante une substance active à l'état dissous ou dispersé.

Par application de ces compositions, par un léger massage sur la peau ou les muqueuses, les microgranulés chargés en substance active fondent progressivement à son contact donnant un système favorable à une action progressive de la substance active sur les tissus ciblés sans agression de celle-ci.

On va maintenant donner à titre d'illustration et sans aucun caractère limitatif des exemples de préparation des microgranulés selon l'invention ainsi que des exemples de compositions pharmaceutiques.

## PROCEDE GENERAL DE PREPARATION DES MICROGRANULES

La substance active (2mg à 2g) et le triglycéride (19,998 à 18g) sont mis à chauffer dans un bécher en verre, éventuellement sous lumière jaune et argon, à une température d'environ 6°C au dessus du point de fusion du triglycéride utilisé, puis placés dans un bain à ultrasons pendant 15min. Le mélange est ensuite versé dans 200ml d'eau stérile à la même température sous agitation constante pendant 3 min. La dispersion obtenue est rapidement refroidie par addition d'eau stérile (450ml à 4°C) sous agitation constante. Les microgranulés chargés de la substance active sont séparés par filtration puis séchés à température ambiante pendant 12 heu-res sous $P_2O_5$ et sous vide (9,45 Pa).

Les microgranulés sont alors soumis à une opération de tamisage en vue d'obtenir des particules de taille requise.

## EXEMPLE 1

Microgranulés chargés d'acide rétinoïque.

Ces microgranulés ont été préparés selon le procédé tel que décrit ci-dessus avec du "SOFTISAN 133®" à l'aide d'un homogénéiseur à 700rpm. La quantité d'acide rétinoïque chargé dans les microgranulés secs à

4

été déterminée par analyse d'absorption UV dans le THF à $\lambda$ max = 348nm ($\varepsilon$ = 47656).

Différents pourcentages d'acide rétinoïque ont été utilisés pour la préparation des microgranulés et les résultats obtenus sont rassemblés dans le tableau suivant:

| Quantité théorique (%) d'acide rétinoïque chargé | Analyse de l'acide rétinoïque (%) dans les microgranulés chargés | % de particules non sphériques | diamètre moyen des particules sphériques ($\mu$m) |
|---|---|---|---|
| 0,5 | 0,507 | 0,73 | 284 |
| 1,0 | 0,920 | 2,8 | 311 |
| 5,0 | 4,770 | 12,0 | 363,3 |
| 10 | 9,800 | 23,4 | 651,6 |

La taille des particules a été déterminée à l'aide d'un analyseur d'image semi-automatique MOP-Videophan (KONTRON)

Aucune trace d'acide rétinoïque solubilisé dans la phase aqueuse, durant le procédé de granulation n'a été détecté par analyse d'absorption UV.

Afin de tester l'efficacité du procédé de séchage des microgranulés, une analyse Karl-Fisher a été réalisée sur des microgranulés chargés avec 0,5% d'acide rétinoïque. La teneur en eau résiduelle dans le microgranulés secs a été d'environ 0,17%.

La stabilité de l'acide rétinoïque dans les microgranulés a été réalisée par analyse UV, la même quantité d'acide rétinoïque (0,4%) a été trouvée au temps O ($T_O$) et après un mois à 4°C.

## EXEMPLE 2

### Microgranulés chargés de 17-valérate de bétaméthasone

Ces microgranulés ont été préparés selon le procédé général tel que décri ci-dessus avec du "SOFTISAN 133®" à l'aide d'un homogénéiseur à 700rpm. La teneur des microgranulés en 17-valérate de bétaméthasone a été contrôlée par dosage HPLC (0,6%).

Après tamisage, les microgranulés présentaient un diamètre de 500μm<∅<800μm.

## EXEMPLE 3

### Microgranulés chargés de 17,21 dipropionate de bétaméthasone

Ces microgranulés ont été préparés selon le procédé général tel que décrit ci-dessus avec du "SOFTISAN 133®" à l'aide d'un homogénéiseur à 700rpm.

La teneur des microgranulés en 17,21-dipropionate de bétaméthasone a été contrôlée par absorbtion UV dans le mélange THF/méthanol à $\lambda$ max = 243nm.

Quantité théorique : 1%
Résultat de l'analyse UV : 0,997%.
Après tamisage, les microgranulés présentaient un diamètre de 200μm<∅<400μm.

## EXEMPLE 4

### Microgranulés chargés d'acide 6-[3-(1-adamantyl)-4-méthoxyphényl]-2 naphtoïque

Ces microgranulés ont été préparés selon le procédé général tel que décrit ci-dessus avec du "SOFTISAN 133®" à l'aide d'un homogénéiseur à 700rpm.

La substance active a été incorporée à un pourcentage théorique de 2% et a été caractérisée par son émission fluorescente (dans le THF) à $\lambda$ = 400nm après excitation à $\lambda$ = 322,5nm.

Les observations microscopiques de l'émission fluorescente des microgranulés chargés montrent que les microparticules des granulés sont de forme sphérique et régulière confirmant la présence homogène de la substance active.

Selon le même mode opératoire que décrit ci-dessus on a également préparé à l'aide de "SOFTISAN 133®" des microgranulés chargés, à un pourcentage théorique de 5%, des substances actives suivantes: (i) minoxidil, (ii) anthraline, (iii) nitrate d'éconazole, (iv) indométhacine et (v) bufexamac. A l'aide de "SOFTISAN 100®" ont été également préparés des microgranulés à un pourcentage théorique de 10% avec le N-benzylphényl-$\alpha$-acétoxyacétamide et à un pourcentage théorique de 2% avec la cinnarizine.

## EXEMPLE 5

### Microgranulés chargés en N-benzylphényl-$\alpha$-acétoxyacétamide

Ces microgranulés ont été préparés par atomisation à l'aide d'un ensemble de pulvérisation sous pression.

Dans un réacteur en acier inoxydable thermostaté à 95 °C on procède, sous azote, à la fusion de 85 gr de "SOFTISAN 138®" et de 15 gr de N-benzylphényl-$\alpha$-acétoxyacétamide. Le mélange fondu est alors poussé jusqu'à la buse à l'aide d'une pression d'azote de 1 bar et la pression de pulvérisation est fixée à 1 bar également. Le gradient de température est préalablement réalisé par introduction d'azote liquide au fond de la cuve.

On obtient ainsi des microgranulés chargés en N-benzylphényl-$\alpha$- acétoxyacétamide d'un diamètre de 5 à 120 μm, le diamètre moyen étant d'environ 30 μm. Après récupération des microgranulés, le taux de principe actif incorporé, dosé par HPLC était d'environ 15 %. Les microgranulés présentaient une forme régulière et l'observation microscopique a révélé l'absence de cristaux libres du principe actif.

## EXEMPLES DE FORMATION

### EXEMPLE 6

Gel aqueux

```
Microgranulés chargés en
17,21-dipropionate de bétaméthasone (0,997%)
                      ®
dans du "SOFTISAN 133" (200µm<φ<400µm).....   5,00g
Carbopol 940..............................   0,475g
NaOH à 10% dans l'eau stérile.............   1,250g
Parahydroxybenzoate de méthyle............   0,075g
Eau déminéralisée stérile.................  93,250g
```

L'activité anti-inflammatoire de ce gel aqueux a été testée dans le modèle d'inflammation provoqué à l'huile de croton, sur l'oreille du cobaye. Elle a été comparée à l'activité d'un gel aqueux de référence contenant la même quantité de 17,21-dipropionate de bétaméthasone à l'état libre. On a constaté que pour le gel aqueux contenant des microgranulés l'activité anti-inflammatoire du 17,21-dipropionate de bétaméthasone était supérieure par rapport au gel aqueux de référence.

### EXEMPLE 7

Gel aqueux

```
Microgranulés chargés en 17-valérate de
                                    ®
bétaméthasone (0,6%) dans du "SOFTISAN 133"
(500µm<φ<800µm)...........................   2,00g
Hydroxypropylcellulose....................   2,00g
Parahydroxybenzoate de méthyle............   0,075g
Tampon pH 7,6 (Na₂HPO₄, NaH₂PO₄)........  95,925g
```

Ce gel aqueux contient 0,012% de 17-valérate de bétaméthasone et sa stabilité a été comparée:
- d'une part à un gel aqueux tamponné à pH 7,6 et contenant la même quantité de 17-valérate de bétaméthasone à l'état libre, et
- d'autre part à une émulsion H/E non-ionique tamponnée à pH 7,6 et contenant également 0,012% de 17-valérate de bétaméthasone à l'état libre.

Le gel aqueux selon l'invention contenant les microgranulés chargés en 17-valérate de bétaméthasone s'est montré beaucoup plus stable que les formulations de référence. On n'a en effet noté l'apparition que d'un très faible pourcentage du produit de réarrangement c'est-à-dire le 21-valérate de bétaméthasone.

### EXEMPLE 8

Poudre anti-inflammatoire

```
Microgranulés chargés en N-benzylphényl-α- acétoxy-
                                    ®
acétamide (15%) dans du "SOFTISAN 138"
(5µm <φ<120µm) ...........................  33,30g
Oxyde de zinc ............................  20,00g
Talc .....................................  46,70g
```

<u>EXEMPLE 9</u>

<u>Onguent hydrophobe</u>

```
Microgranulés chargés en N-benzyl-α-acétoxyacétamide (15%)
dans du "SOFTISAN 138"® (5µm<φ<30µm) .........    33g
Silice vendue par la Société DEGUSSA sous
la dénomination de "AEROSIL 200"® ...........    2g
Huile de Silicone (polydiméthylsiloxane)
vendue par la Société RHONE-POULENC sous la
dénomination de "RHODORSIL®70047 V 100.000" ..   1g
Huile de Silicone (polydiméthylsiloxane) vendue
par la Société RHONE-POULENC sous la
dénomination de "RHODORSIL®70047 V 300" ......  64g
```

## Revendications

1. Microgranulés chargés en une substance thérapeutiquement active sur les désordres cutanés et fondant par application sur la peau, caractérisés par le fait qu'ils sont essentiellement formés, par granulation de ladite substance active, à l'aide d'au moins un triglycéride d'acides gras saturés, linéaires ou ramifiés ayant de 10 à 22 atomes de carbone, ayant un point de fusion compris entre 30 et 50°C, lesdits microgranulés ayant un diamètre moyen compris entre environ 5µm et 800µm.

2. Microgranulés selon la revendication 1, caractérisés par le fait qu'ils ont un diamètre moyen compris entre 50 et 500µm.

3. Microgranulés selon la revendication 1 ou 2, caractérisés par le fait que les acides gras saturés du triglycéride proviennent d'acides gras végétaux naturels

4. Microgranulés selon l'une quelconque des revendications précédentes, caractérisés par le fait que le triglycéride d'acides gras saturés a un indice d'hydroxyle inférieur ou égal à 15 et un indice d'iode inférieur ou égal à 3.

5. Microgranulés selon l'une quelconque des revendications précédentes, caractérisés par le fait qu'ils contiennent de 0,01 à 10% en poids de la substance active.

6. Microgranulés selon l'une quelconque des revendications précédentes, caractérisés par le fait que la substance active est la vitamine A, l'acide rétinoïque et ses dérivés, les vitamines E et F et leurs dérivés, l'acide 6-[3-(1-adamantyl-4) méthoxyphényl] naphtoïque-2 et ses analogues, l'acide 2-(5,6,7,8-tétrahydro-5,5,8,8- tétraméthyl-2-naphtyl)-6-benzo (b) thiophène carboxylique et ses analogues, l'acide 4- [3-(1-adamantyl)-4-méthoxybenzamido ] benzoïque et ses analogues, les corticostéroïdes tels que le 17-valérate de bétaméthasone ou le 17,21-dipropionate de bétaméthasone, les anti-inflammatoires non stéroïdiens tels que l'indométhacine, le bufexamac, le N-benzylphényl- α acétoxyacétamide ; ou d'autres dérivés du N-benzylmandélamide tels que le nicotinate de N-benzylmandélamide, le benzoate de N-benzylmandélamide, l'acétate de N-(p-chloro)benzylmandélamide ; l'acétate de N-(p-méthyl)benzylmandélamide, l'acétate de N-méthyl, N-benzylmandélamide, l'O-éthoxy-carbonyl-N-benzylmandélamide ; le N-phénéthyl-diphényl-acétoxyacétamide ; les dérivés d'acides gras polyinsaturés tels que l'acide éicosatriynoïque, les antihistaminiques tels que la prométhazine ou la cinnarizine, les antiprurigineux tels que l'acide palmitoyl-collagénique, les antifongiques tels que les dérivés d'éconazole ou les dérivés de pyrithione, les antipsoriasiques tels que l'anthraline et ses dérivés, les antibactériens tels que les sulfamides, les antibiotiques tels l'érythromycine, les antiseptiques tels que les parahydroxybenzoates de propyle ou de benzyle, les antiviraux et antiherpétiques tels que la vidarabine, les anesthésiques tels que la benzocaïne, les kératolytiques tels que l'acide salicylique ou les goudrons, ou les peroxydes tels que le peroxyde de benzoyle, les agents vasodilatateurs tels que les esters de l'acide nicotinique, les agents vasculoprotecteurs ou veinotoniques tels que l'extrait de marron d'inde, les agents anticouperose, les agents aidant à la cicatrisation, les agents anti-chute ou favorisant la repousse des cheveux, tels que le minoxidil.

7. Composition pharmaceutique, caractérisée par le fait qu'elle contient dans un véhicule approprié des microgranulés chargés en une substance active tels que revendiqués selon l'une quelconque des revendications 1 à 6.

8. Composition selon la revendication 7, caractérisée par le fait qu'elle contient les microgranulés chargés en substance active, en une proportion comprise entre 1 et 50% par rapport au poids total de la composition.

9. Composition selon l'une quelconque des revendications 7 et 8, caractérisée par le fait qu'elle se présente sous forme d'une lotion, d'un gel aqueux hydroalcoolique ou hydroglycolique, d'une formulation à base de silicone, d'une poudre ou encore d'une pommade.


**Patentansprüche**

1. Mikrokügelchen, die mit einer bei Hautstörungen therapeutisch aktiven Substanz beladen sind und bei Aufbringung auf die Haut schmelzen, dadurch gekennzeichnet, daß sie im wesentlichen durch Granulation der aktiven Substanz mit Hilfe wenigstens eines Triglycerids von gesättigten linearen oder verzweigten Fettsäuren mit 10 bis 22 Kohlenstoffatomen, das einen Schmelzpunkt zwischen 30 und 50 °C aufweist, gebildet werden, wobei die Mikrokügelchen einen mittleren Durchmesser zwischen etwa 5 μm und 800 μm aufweisen.

2. Mikrokügelchen nach Anspruch 1, dadurch gekennzeichnet, daß sie einen mittleren Durchmesser zwischen 50 und 500 μm aufweisen.

3. Mikrokügelchen nach Anspruch 1 oder 2, dadurch gekennzeichnet, daß die gesättigten Fettsäuren des Triglycerids von natürlichen pflanzlichen Fettsäuren abstammen.

4. Mikrokügelchen nach einem der vorangehenden Ansprüche, dadurch gekennzeichnet, daß das Triglycerid der gesättigten Fettsäuren eine Hydroxylzahl kleiner als oder gleich 15 und eine Iodzahl kleiner als oder gleich 3 aufweist.

5. Mikrokügelchen nach einem der vorangehenden Ansprüche, dadurch gekennzeichnet, daß sie 0,01 bis 10 Gew.-% der aktiven Substanz enthalten.

6. Mikrokügelchen nach irgendeinem der vorangehenden Ansprüche, dadurch gekennzeichnet, daß die aktive Substanz Vitamin A, Retinolsäure und ihre Derivate, die Vitamine E und F und ihre Derivate, 6-[3-(1-Adamant-4-yl-)methoxyphenyl-]2-naphtholsäure und ihre Analoge, 2-(5,6,7,8-Tetrahydro-5,5,8,8-tetramethyl-2-naphthyl-) 6-benzo(b)thiophencarbonsäure und ihre Analoge, 4-[3-(1-Adamantyl-) 4-methoxybenzamido-] benzoesäure und ihre Analoge, Corticosteroide wie das 17-Valerat von Betamethason oder das 17,21-Dipropionat von Betamethason, entzündungshemmende Nicht-Steroide wie Indometacin, Bufexamac, N-Benzylphenyl-α-acetoxyacetamid oder andere Derivate von N-Benzylmandelamid wie beispielsweise das Nicotinat des N-Benzylmandelamids, das Benzoat des N-Benzylmandelamids, das Acetat des N-(p-Chlor-) benzylmandelamids, das Acetat des N-(p-Methyl-) benzylmandelamids, das Acetat des N-Methyl-N-benzylmandelamids, O-Ethoxycarbonyl-N-benzylmandelamid, N-Phenethyldiphenylacetoxyacetamid, Derivate mehrfach ungesättigter Fettsäuren wie beispielsweise Eicosatriensäure, Antihistaminika wie beispielsweise Promethazin oder Cinnarizin, Antijuckmittel wie beispielsweise Palmitoylkollagensäure, Anti-Pilzmittel wie beispielsweise Derivate des Econazols oder Derivate des Pyrithions, Anti-Psoriasismittel wie beispielsweise Anthralin und seine Derivate, antibakterielle Mittel wie beispielsweise Sufamide, Antibiotika wie beispielsweise Erythromycin, Antiseptika wie beispielsweise Propyl- oder Benzylparahydroxybenzoat, antivirale Mittel und Anti-Herpesmittel wie beispielsweise Vidarabin, Anästhetika wie beispielsweise Benzocain, Keratolytika wie beispielsweise Salicylsäure oder Teere oder Peroxide wie beispielsweise Benzoylperoxid, vasodilatorisch wirkende Mittel wie bei-

spielsweise Ester der Nicotinsäure, gefäßschützende oder venenkräftigende Mittel wie beispielsweise Roßkastanienextrakt, Anti-Couperosemittel, die Narbenbildung unterstützende Mittel, Mittel gegen Haarausfall oder den Haarwuchs fördernde Mittel wie beispielsweise Minoxidil ist/sind.

7. Pharmazeutische Zubereitung, dadurch gekennzeichnet, daß sie in einem geeigneten Träger Mikrokügelchen enthält, die mit einer aktiven Substanz beladen sind, wie sie in einem der Ansprüche 1 bis 6 beansprucht ist.

8. Zubereitung nach Anspruch 7, dadurch gekennzeichnet, daß sie die mit einer aktiven Substanz beladenen Mikrokügelchen in einer Menge zwischen 1 und 50 % enthält, bezogen auf das Gesamtgewicht der Zubereitung.

9. Zubereitung nach einem der Ansprüche 7 oder 8, dadurch gekennzeichnet, daß sie in Form einer Lotion, eines wäßrig-hydroalkoholischen oder -hydroglykolischen Gels, einer Formulierung auf der Basis von Silicon, eines Puders oder einer Pomade vorliegt.

## Claims

1. Microgranules loaded with a substance which is therapeutically active against skin disorders and which melts on application to the skin, characterised in that they are essentially formed by granulation of the said active substance with the aid of at least one triglyceride of saturated, linear or branched fatty acids having from 10 to 22 carbon atoms and a melting point of between 30 and 50°C, the said microgranules having an average diameter of between approximately 5 µm and 800 µm.

2. Microgranules according to Claim 1, characterised in that they have an average diameter of between 50 and 500 µm.

3. Microgranules according to Claim 1 or 2, characterised in that the saturated fatty acids of the triglyceride originate from natural plant fatty acids.

4. Microgranules according to any one of the preceding claims, characterised in that the triglyceride of saturated fatty acids has a hydroxyl value not exceeding 15 and an iodine value not exceeding 3.

5. Microgranules according to any one of the preceding claims, characterised in that they contain from 0.01 to 10% by weight of the active substance.

6. Microgranules according to any one of the preceding claims, characterised in that the active substance is vitamin A, retinoic acid and its derivatives, vitamins E and F and their derivatives, 6-[3-(1-adamantyl-4)methoxyphenyl]-2-naphthoic (sic) acid and its analogues, 2-(5,6,7,8-tetrahydro-5,5,8,8-tetramethyl-2-naphtho)-6-benzo[b]thiophenecarboxylic acid and its analogues, 4-[3-(1-adamantyl)-4-methoxybenzamido]benzoic acid and its analogues, corticosteroids such as betamethasone 17-valerate of betamethasone 17,21-dipropionate, non-steroidal anti-inflammatories such as indomethacin, bufexamac, N-benzylphenyl-$\alpha$-acetoxyacetamide; or other N-benzylmandelamide derivatives such as N-benzylmandelamide nicotinate, N-benzylmandelamide benzoate, N-(p-chloro)benzylmandelamide acetate; N-(p-methyl) benzylmandelamide acetate, N-methyl-N-benzylmandelamide acetate, O-ethoxycarbonyl-N-benzylmandelamide; N-(phenethyl)diphenylacetoxyacetamide; derivatives of polyunsaturated fatty acids such as icosatriynoic acid, antihistaminics such as promethazine or cinnarizine, antipruritics such as palmitoylcollagenic acid, antifungals such as econazole derivatives of pyrithione derivatives, antipsoriatics such as anthralin and its derivatives, antibacterials such as sulphonamides, antibiotics such as erythromycin, antiseptics such as propyl or benzyl para-hydroxybenzoates, antivirals and antiherpetics such as vidarabine, anaesthetics such as benzocaine, keratolytics such as salicylic acid or tars, peroxides such as benzoyl peroxide, vasodilator agents such as nicotinic acid esters, vasoprotective or veinotonic agents such as horsechestnut extract, antirosacea agents, agents assisting cicatrisation or agent combating hair loss of promoting hair regrowth, such as minoxidil.

7. Pharmaceutical composition, characterised in that it contains, in a suitable vehicle, microgranule loaded with an active substance as are claimed according to any one of Claims 1 to 6.

8. Composition according to Claim 7, characterised in that it contains the microgranules loaded with active substance, in a proportion of between 1 and 50% relative to the total weight of the composition.

9. Composition according to either of Claims 7 and 8, characterised in that it takes the form of a lotion, an aqueous-alcoholic gel or a water/glycol-based gel, a silicone-based formulation, a powder or alternatively an ointment.